# EUROPÄISCHE PATENTANMELDUNG

(11) **EP 2 774 869 A1**
(43) Veröffentlichungstag der Anmeldung: **10.09.2014**
(21) Anmeldenummer: 14000675.0
(22) Anmeldetag: 26.02.2014
(51) Int. Cl.: B65D 45/24, E05C 19/14, A61B 19/02

(54) **Behälterverschluss**

(30) Priorität: 08.03.2013 DE 202013002202 U
(71) Anmelder: Innovations Medical GmbH, 78532 Tuttlingen (DE)
(72) Erfinder: Kreidler, Winfried, 78532 Tuttlingen (DE); Kreidler, Jochen, 78532 Tuttlingen (DE)
(74) Vertreter: Binner, Bernhard

(57) **Zusammenfassung**

Die Erfindung betrifft einen Behälterverschluss (4) zum Verriegeln Sterilbehälters (1), mit einer Verschlussklappe (5), welche am Behälterdeckel (2) um eine Schwenkachse (12) aus einer geöffneten Schwenkstellung in eine geschlossene Schließstellung schwenkbar gelagert ist und mit einem am freien Ende der Verschlussklappe (5) angeordneten Spannhebel (14), welcher aus einer geöffneten Ausgangsstellung in eine geschlossene Endstellung um eine Drehachse (15) an der Verschlussklappe (5) drehbar gelagert ist und, mit einem am Unterteil (3) des Sterilbehälters (1) nach außen vorstehenden Kupplungselement (19), wobei der Spannhebel (14) ein Formschlusselement (21) aufweist, welches beim Schließvorgang mit einem Formteil (20) des Kupplungselementes (19) in Eingriff bringbar ist. Um insbesondere den Schließvorgang zu vereinfachen, ist ein erstes zwischen der Verschlussklappe (5) und dem Spannhebel (14) wirksames Fixierelement (55) vorgesehen, durch welches die geöffnete Ausgangsstellung des Spannhebels (14) relativ zur Verschlussklappe (5) derart definiert ist, dass der Spannhebel (14) während der Schließbewegung der Verschlussklappe (5) zwangläufig mit seinem Formschusselement (21) mit dem Formteil (20) des Kupplungselementes (19) in Eingriff gelangt.

## Beschreibung

Die Erfindung betrifft einen nach dem Übertotpunktprinzip arbeitenden Behälterverschluss zum Verriegeln eines aus einem Behälterdeckel und einem kastenartigen Unterteil bestehenden Sterilbehälters, mit einer Verschlussklappe, welche mit ihrem einen Ende am Behälterdeckel um eine Schwenkachse aus einer geöffneten Schwenkstellung in eine geschlossene Schließstellung schwenkbar gelagert ist und mit einem am anderen, freien Ende der Verschlussklappe angeordneten Spannhebel, welcher aus einer geöffneten Ausgangsstellung in eine geschlossene Endstellung um eine Drehachse an der Verschlussklappe drehbar gelagert ist und, mit einem am Unterteil des Sterilbehälters nach außen vorstehenden Kupplungselement, wobei der Spannhebel ein Formschlusselement aufweist, welches beim Schließvorgang mit einem Formteil des Kupplungselementes in Eingriff bringbar ist.

Behälterverschlüsse der gattungsgemäßen Art sind schon seit langem bekannt. Hierzu sei beispielhaft auf die US 2 867 863 A, die US 3 892 434 A, die US 3 618 995 A sowie die DE 20 2012 002 487.6 U1 verwiesen.

Diesen Behälterverschlüssen ist gemeinsam, dass diese nach dem Übertotpunktprinzip arbeiten und eine Art Verschlussklappe aufweisen, welche mit ihrem einen Ende, beispielsweise am Behälterdeckel eines Sterilbehälters oder auch eines anderweitig ausgestalteten Behälters, um eine Schwenkachse schwenkbar gelagert ist. Dabei kann diese Verschlussklappe aus einer geöffneten Schwenkstellung in eine geschlossene Schließstellung geschwenkt werden. Am freien Ende der Verschlussklappe ist eine Art Spannhebel vorgesehen, welcher um eine Drehachse an diesem Ende der Verschlussklappe drehbar gelagert ist. Dieser Spannhebel weist dabei, ausgehend von der Drehachse, eine Art Betätigungsabschnitt auf, welchem bezüglich der Drehachse im Wesentlichen diametral gegenüberliegend ein Formschlusselement zugeordnet ist.

Durch gleichzeitiges Schwenken der Verschlussklappe und Drehen des Spannhebels kann dieses Formschlusselement mit einem Formteil eines Kupplungselementes in Eingriff gebracht werden, welches beispielsweise am Unterteil des Sterilbehälters nach außen vorstehend angeordnet ist. Dabei wird der Spannhebel aus einer "beliebigen" Ausgangsstellung zunächst derart ausgerichtet, dass dessen Formschlusselement beim Schwenken der Verschlussklappe um die Schwenkachse tatsächlich mit dem Formteil des Kupplungselementes in Eingriff gebracht werden kann.

Durch weiteres Drehen des Spannhebels bleibt die räumliche Position des Formschlusselementes aufgrund des Eingriffes mit dem Formteil unverändert, so dass bei einem weiteren Drehen des Spannhebels gleichzeitig die Verschlussklappe um die Schwenkachse in ihre Schließstellung gebracht wird. Nach Überschreiten des "Totpunktes" gelangt die Drehachse des Spannhebels in eine Position, in welcher diese Drehachse beispielsweise zur Seitenwand des Unterteils des Sterilbehälters einen geringeren Abstand aufweist als die "Formschlussverbindung" zwischen dem Formteil des Kupplungselementes und dem Formschlusselement des Spannhebels. Damit wird ein solcher Behälterverschluss in dieser geschlossenen "Spannstellung" nach dem Übertotpunktprinzip selbstständig in seiner geschlossenen Stellung gehalten.

Aufgrund der freien Drehbarkeit des Spannhebels relativ zur Verschlussklappe, wird das Ansetzen des Formschlusselementes des Spannhebels am Formteil des Kupplungselementes erschwert. So ist häufig zu beobachten, dass der Spannhebel mit seinem Formschlusselement zunächst auf das Unterteil des Sterilbehälters ausgerichtet wird. anschließend wird die Verschlussklappe in Richtung des Unterteils geschwenkt, bis das Formschlusselement an der Seitenwand des Unterteils ansteht. Durch anschließendes Drehen des Spannhebels um dessen Drehachse, gleitet nunmehr das Formschlusselement entlang der Seitenwand, bis dieses mit dem Formteil des Kupplungselementes in Eingriff steht. Aufgrund dieser Handhabung findet zwangsläufig eine Beschädigung der Behälterwand statt.

Demgemäß liegt der Erfindung die Aufgabe zugrunde, einen Behälterverschluss der gattungsgemäßen Art derart zu verbessern, dass eine solche Beschädigung sicher ausgeschlossen ist.

Die Aufgabe wird erfindungsgemäß zusammen mit den Merkmalen des Oberbegriffes des Anspruches 1 dadurch gelöst, dass ein erstes zwischen der Verschlussklappe und dem Spannhebel wirksames Fixierelement vorgesehen ist, durch welches die geöffnete Ausgangsstellung des Spannhebels relativ zur Verschlussklappe derart definiert ist, dass der Spannhebel während der Schließbewegung der Verschlussklappe zwangläufig mit seinem Formschusselement mit dem Formteil des Kupplungselementes in Eingriff gelangt.

Aufgrund der erfindungsgemäßen Ausgestaltung wird eine Beschädigung der Seitenwand oder eines darauf aufgesetzten Teiles der Seitenwand sicher verhindert. So ist erfindungsgemäß vorgesehen, dass der Spannhebel eine definierte geöffnete Ausgangsstellung einnimmt, in welcher dieser zwangsläufig mit seinem Formschlusselement mit dem Formteil des Kupplungselementes in Eingriff gelangt, sofern die Verschlussklappe in Richtung ihrer Schließstellung geschwenkt wird. Das heißt letztendlich, dass in Öffnungsrichtung der Spannhebel nur begrenzt verschwenkbar ist. Hierzu ist ein zwischen der Verschlussklappe und dem Spannhebel wirksames Fixierelement vorgesehen. Ein solches Fixierelement kann dabei als Anschlag oder auch als Rastelement ausgestaltet sein.

Vorzugsweise ist hier gemäß Anspruch 2 vorgesehen, dass das Fixierelement als Anschlag ausgebildet ist, welches am Spannhebel oder an der Verschlussklappe feststehend angeordnet ist. Ist dieser Anschlag am Spannhebel angeordnet, so wird dessen Drehwinkel in Richtung "öffnen" begrenzt, in dem dieser Anschlag in einer vordefinierten Drehposition des Spannhebels relativ zur Verschlussklappe an der Verschlussklappe ansteht.

Ist dieser Anschlag entsprechend an der Verschlussklappe angeordnet, so läuft der Spannhebel in Öffnungsrichtung in einer vorbestimmten Winkelstellung gegen diesen Anschlag der Verschlussklappe, so dass ebenfalls dessen geöffnete Relativstellung zur Verschlussklappe präzise definiert ist.

Weiter kann gemäß Anspruch 3 vorgesehen sein, dass zur Fixierung der relativen Ausgangsstellung des Spannhebels zur Verschlussklappe ein Rastelement vorgesehen ist, mit welchem der Spannhebel in seiner Ausgangsstellung rastend in Eingriff steht. Dieses Rastelement kann dabei alternativ zum Anschlag vorgesehen sein. Vorzugsweise ist jedoch dieses Rastelement zusätzlich zum Anschlagelement vorgesehen. Denn ein solches Rastelement birgt die Gefahr, je nach konkreter Ausgestaltung, dass der Spannhebel in Öffnungsrichtung am Rastelement bei unsachgemäßer Handhabung überrasten kann und somit das Formschlusselement in eine Lage gebracht werden kann, in welcher dieses bei der Schließbewegung der Verschlussklappe in Schließrichtung mit der Seitenwand eines Unterteils eines Sterilbehälters in Kontakt kommt.

Weitere vorteilhafte Ausgestaltungen der Erfindung sind den weiteren Unteransprüchen zu entnehmen.

So kann weiter gemäß Anspruch 4 vorgesehen sein, dass das Rastelement als Rastkugel ausgebildet ist, welche in einem separaten Gewindezapfen federelastisch nachgiebig angeordnet ist und, dass der Gewindezapfen in ein Innengewinde der Verschlussklappe parallel zur Schwenkachse der Verschlussklappe verlaufend angeordnet ist und, dass der Spannhebel ein Formschlusselement aufweist, mit welchem die Rastkugel rastend in Eingriff bringbar ist. Dementsprechend gelangt die Rastkugel in der geöffneten Ausgangsstellung des Spannhebels mit dem Formschlusselement des Spannhebels formschlüssig in Eingriff, so dass die Ausgangsstellung fixiert ist. Dementsprechend kann der Spannhebel eine Vertiefung, einen Durchbruch oder auch einen zur Rastkugel hin vorstehenden Eingriffssteg im Sinne eines Formschlusselementes aufweisen, welches in der Ausgangsstellung des Spannhebels relativ zur Verschlussklappe mit der Rastkugel des Rastelementes in Eingriff steht.

Weiter kann gemäß Anspruch 5 vorgesehen sein, dass die geschlossene Endstellung des Spannhebels relativ zur Verschlussklappe durch ein zweites Fixierelement derart definiert ist, dass der Spannhebel in seiner geschlossenen Endstellung nicht am Unterteil anliegt. Durch diese Ausgestaltung wird der Drehwinkel des Spannhebels relativ zur Verschlussklappe in "Schließrichtung" begrenzt. Auch durch diese Maßnahme wird sicher verhindert, dass das Unterteil, insbesondere dessen Seitenwand oder ein Aufbauteil der Seitenwand, mit dem Spannhebel in Kontakt kommt, so dass auch hier keinerlei Beschädigung auftreten kann.

Hierzu kann gemäß Anspruch 6 auch das zweite Fixierelement als Anschlag ausgebildet sein, welcher am Spannhebel oder an der Verschlussklappe feststehend angeordnet ist.

Anhand der Zeichnung wird nachfolgend beispielhaft eine Ausführungsvariante der Erfindung näher erläutert. Es zeigt:
- Fig. 1: eine perspektivische Teilansicht eines Sterilbehälters mit einem Behälterverschluss;
- Fig. 2: eine perspektivische Explosionsdarstellung der einzelnen Bauteile des Behälterverschlusses;
- Fig. 3: eine Seitenansicht des Behälterverschlusses in einer geöffneten Ausgangsstellung;
- Fig. 4: den Behälterverschluss aus Fig. 3 in Seitenansicht in einer Zwischenstellung, in welcher das Formschlusselement des Spannhebels mit dem Formteil des Kupplungselementes in Eingriff gelangt;
- Fig. 5: die Seitenansicht aus Fig. 4 mit dem vollständig geschlossenen Behälterverschluss;
- Fig. 6: einen vergrößerten Ausschnitt VI aus Fig. 5.

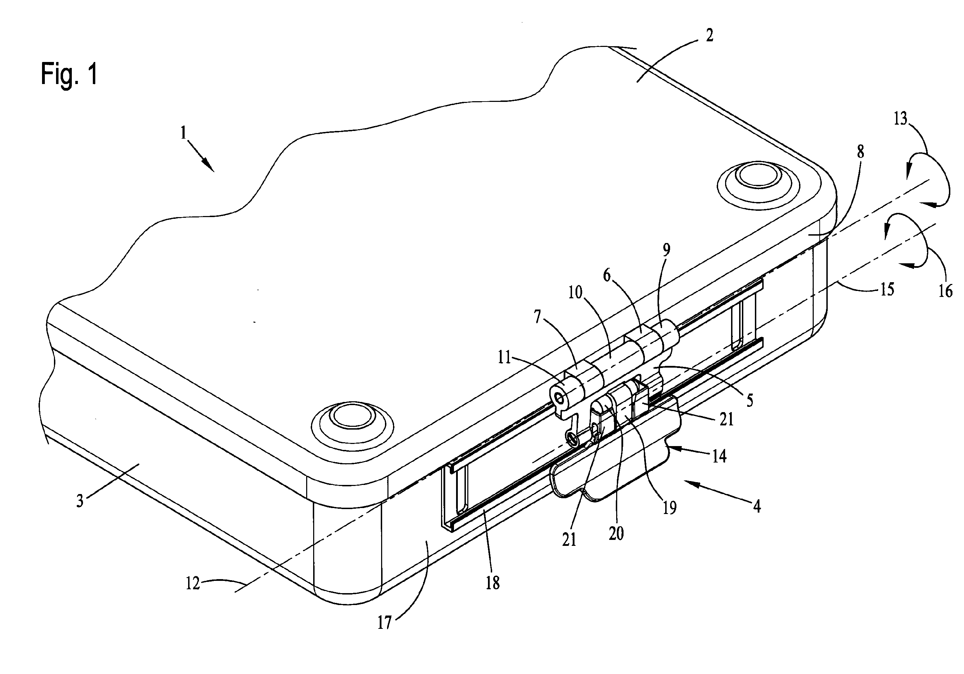

Fig. 1 zeigt eine perspektivische Teilansicht eines Sterilbehälters 1, welcher bei dem dargestellten Ausführungsbeispiel einen oberen Behälterdeckel 2 aufweist. Dieser Behälterdeckel 2 ist auf ein kastenartiges Unterteil 3 aufgesetzt. Im geschlossenen Zustand sind der Behälterdeckel und das Unterteil mittels einer umlaufenden, elastisch nachgiebigen Dichtung nach außen hin abgedichtet (in der Zeichnung nicht dargestellt).

Des Weiteren ist aus Fig. 1 erkennbar, dass der Sterilbehälter 1 einen Behälterverschluss 4 aufweist, welcher einerseits mit dem Behälterdeckel 2 und andererseits mit dem Unterteil 3 in Verbindung steht. Dieser Behälterverschluss 4 weist eine Verschlussklappe 5 auf, welche über zwei Lageraugen 6 und 7 des Behälterdeckels 2 an dessen vorderer Stirnleiste 8 schwenkbar gelagert ist. Zur schwenkbaren Lagerung an den beiden Lageraugen 6 und 7 bildet die Verschlussklappe 5 beim dargestellten Ausführungsbeispiel insgesamt drei Schwenklager 9, 10 und 11, so dass die Verschlussklappe 5 um eine Schwenkachse 12 in Richtung des Doppelpfeils 13 relativ zum Behälterdeckel 2 schwenkbar ausgebildet ist.

In dem freien, den Schwenklagern 9, 10 und 11 gegenüberliegenden Endbereich, ist an der Verschlussklappe 5 ein Spannhebel 14 angeordnet, welcher um eine Drehachse 15 in Richtung des Doppelpfeils 16 relativ zur Verschlussklappe 5 drehbar ausgebildet ist.

Unterhalb der Stirnleiste 8 des Behälterdeckels 2 ist auf der vorderseitigen Frontwand 17 des Unterteils 3 eine Montageleiste 18 vorgesehen, welche ein zentrales, nach außen vorstehendes Kupplungselement 19 aufweist. In diesem in Fig. 1 dargestellten geschlossenen Zustand des Behälterverschlusses 4 steht der Spannhebel 14 mit einem quer verlaufenden Formteil in Form eines Sperrzapfens 20 des Kupplungselementes 19 über ein zweiteiliges Formschlusselement 21 in Wirkverbindung.

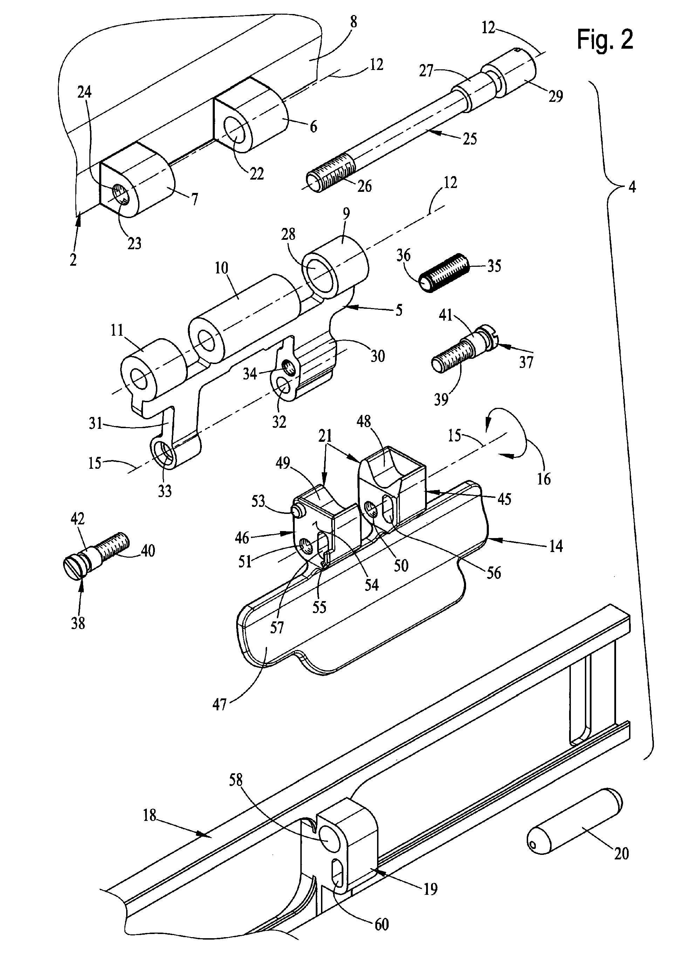

Fig. 2 zeigt bezüglich des Behälterverschlusses 4 eine perspektivische Explosionsdarstellung der zugehörigen Bauteile.

Aus Fig. 2 ist der Behälterdeckel 2 mit seiner nach unten gerichteten Stirnleiste 8 in perspektivischer Teilansicht erkennbar. An dieser Stirnleiste 8 sind die beiden Lageraugen 6 und 7 vorgesehen. Jedes dieser Lageraugen 6, 7 weist eine Querbohrung 22 bzw. 23 auf, wobei bei der dargestellten Ausführungsvariante die Querbohrung 22 des Lagerauges 6 einen größeren Durchmesser aufweist als die Querbohrung 23 des Lagerauges 7. Des Weiteren ist die Querbohrung 23 des Lagerauges 7 mit einem Innengewinde 24 versehen, in welches eine Lagerschraube 25 mit einem entsprechenden Außengewinde 26 einschraubbar ist.

Diese Lagerschraube 25 weist in ihrem, dem Außengewinde 26 gegenüber liegenden Endbereich einen radial erweiterten Lagerzylinder 27 auf, mit welchem die Lagerschraube 25 passend in die Querbohrung 22 des Lagerauges 6 einsetzbar ist. Im montierten Zustand definiert somit die Lagerschraube 25 die Schwenkachse 12 zur schwenkbaren Lagerung der Verschlussklappe 5 am Behälterdeckel 2. Diese Schwenkachse 12 ist in Bezug auf die Schwenklager 9, 10 und 11 auch für die Verschlussklappe 5 in der Zeichnung dargestellt.

Weiter ist aus Fig. 2 erkennbar, dass das Schwenklager 9 eine im Durchmesser größer ausgebildete Lagerbohrung 28 aufweist, welche im montierten Zustand einen radial erweiterten Schraubenkopf 29 der Lagerschraube 25 aufnimmt.

Weiter ist aus Fig. 2 erkennbar, dass die Verschlussklappe 5 in der dargestellten räumlichen Orientierung zwei nach unten gerichtete Lagerzungen 30 und 31 bildet. Die Lagerzunge 30 ist in ihrer Breite größer ausgebildet als die Lagerzunge 31, wobei beide Lagerzungen 30 und 31 in ihrem jeweils unteren Endbereich eine Lagerbohrung 32 bzw. 33 aufweisen. Oberhalb der Lagerbohrung 32 weist die Lagerzunge 30 ein durchgehendes parallel zur Schwenkachse 12 verlaufendes Innengewinde 34 auf, in welches ein Gewindezapfen 35 einschraubbar ist. Dieser Gewindezapfen 35 weist auf seiner einen Stirnseite eine federbelastete Rastkugel 36 auf, welche im montierten Zustand im Innengewinde 34 zur zweiten Lagerzunge 31 hin über die erste Lagerzunge 30 vorsteht.

Die beiden Lagerbohrungen 32 und 33 dienen zur drehbaren Aufnahme zweier Lagerschrauben 37 und 38, welche mit einem entsprechenden Gewindeabschnitt 39 und 40 mit dem Spannhebel 14 feststehend verbindbar sind. Zur drehbaren Lagerung dieser Lagerschrauben 37 und 38 in den Lagerbohrungen 32 und 33 weisen diese jeweils einen radial erweiterten Lagerzylinder 41 bzw. 42 auf.

Weiter ist aus Fig. 2 erkennbar, dass das zweiteilige Formschlusselement 21 zwei Sperrelemente 45 und 46 bildet, welche in der in Fig. 2 dargestellten "geschlossenen" Schließstellung des Spannhebels 14 vertikal nach oben gerichtet an dessen Betätigungselement 47 angeordnet sind. Oberseitig weist jedes Sperrelement 45 bzw. 46 jeweils eine V-förmige ausgebildete Eingriffsnut 48 bzw. 49 auf. Unterhalb dieser Eingriffsnuten 48, 49, im Wesentlichen direkt oberhalb des Betätigungselementes 47, ist jedes Sperrelement 45 bzw. 46 mit einem Innengewinde 50 bzw. 51 versehen, welche zusammen die Drehachse 15 definieren. In diese Innengewinde 50 bzw. 51 ist jeweils eine der Lagerschrauben 37 bzw. 38 außenseitig einschraubbar, so dass über diese Lagerschrauben 37 und 38 der Spannhebel 14 um die Drehachse 15 und damit um die Lagerbohrungen 32 und 33, welche im montierten Zustand ebenfalls die Drehachse 15 definieren, frei drehbar ist.

Weiter ist aus Fig. 2 erkennbar, dass das eine Sperrelement 46 ein in Form eines Anschlages 53 ausgebildetes zweites Fixierelement aufweist, welches seitlich vorstehend im "hinteren" Bereich der Seitenwand 54 des Sperrelementes 46 angeordnet ist. Im Bereich der vorderen, unteren Endkante bildet das Sperrelement 46 ein weiteres, erstes Fixierelement in Form eines weiteren Anschlages 55. Durch diese beiden Fixierelemente 53 und 55 wird der maximal mögliche Drehwinkel in Richtung des Doppelpfeils 16 des Spannhebels 14 relativ zur Verschlussklappe 5 begrenzt.

Des Weiteren ist aus Fig. 2 erkennbar, dass die beiden Sperrelemente 45 und 46 im Bereich ihrer Vorderkante jeweils einen langlochartigen Durchbruch 56 bzw. 57 aufweisen. Diese beiden Durchbrüche 56 und 57 dienen im "geschlossenen" Sperrzustand des Behälterverschlusses 4 zur Lagesicherung, indem beispielsweise ein Sicherungsbolzen hindurch gesteckt wird.

Im geöffneten Zustand, in welchem sich der Spannhebel 14 in einer geöffneten Ausgangslage relativ zur Verschlussklappe 5 befindet, steht der Durchbruch 56 "außenseitig" formschlüssig mit der Rastkugel 36 des Gewindezapfens 35 in Eingriff, so dass durch diese Rastverbindung diese Aufgangslage fixiert ist.

Des Weiteren ist aus Fig. 2 noch die Montageleiste 18 in perspektivischen Teilansicht erkennbar. Diese Montageleiste 18 trägt das Kupplungselement 19, welches nach außen vorstehend an der Montageleiste 18 angeformt ist. Alternativ zu dieser Ausgestaltung könnte das Kupplungselement 19 auch direkt am Unterteil 3 des Sterilbehälters aus Fig. 1 angeordnet sein. Dieses Kupplungselement 19 weist beim dargestellten Ausführungsbeispiel eine Querbohrung 58 auf, in welche das Formteil in Form des Sperrzapfens 20 feststehend einsetzbar ist. Dieser Sperrzapfen 20 kann dabei in die Querbohrung 58 beispielsweise eingepresst sein.

Unterhalb der Querbohrung 58 ist im Kupplungselement 19 ein langlochartiger Durchbruch 60 vorgesehen, welcher in Verbindung mit den langlochartigen Durchbrüchen 56 und 57 der beiden Sperrelemente 45 und 46 zur Lagesicherung der geschlossenen Schließposition des Behälterverschlusses 4 dient.

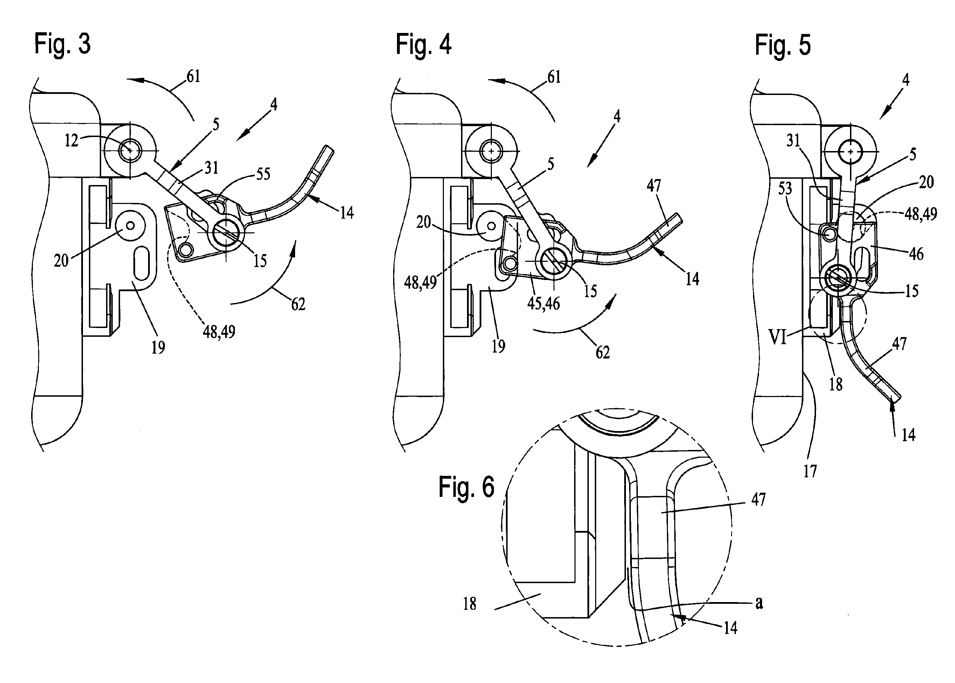

In den teilweisen Seitenansichten der Fig. 3, 4 und 5 wird nachfolgend die prinzipielle Funktionsweise des Behälterverschlusses 4 näher erläutert.

So zeigt die Seitenansicht der Fig. 3 die Verschlussklappe 5 in einer geöffneten Ausgangsposition, in welcher diese um die Schwenkachse 12 in Richtung des Pfeiles 61 geschwenkt ist. In dieser hier beispielhaft dargestellten, geöffneten Schwenkposition, befindet sich die Drehachse 15 zusammen mit dem Spannhebel 14 in einem Abstand zum Kupplungselement 19, welcher derart bemessen ist, dass die beiden Eingriffsnuten 48 und 49 nicht mit dem Sperrzapfen 20 in Eingriff stehen.

In dieser "Ausgangsposition" der Verschlussklappe 5 befindet sich der Spannhebel 14 in einer in Richtung des Pfeiles 62 gedrehten, geöffneten Ausgangsstellung relativ zur Verschlussklappe 5. Diese Ausgangsstellung wird durch den ersten Anschlag 55 des Spannhebels 14 definiert, welcher an der Lagerzunge 31 der Verschlussklappe 5 ansteht. Wie bereits zu Fig. 2 erwähnt, wird diese relative, geöffnete Ausgangsstellung des Spannhebels 14 zur Verschlussklappe 5 durch die Rastkugel 36 des Gewindezapfens 35 fixiert, welcher in Fig. 3 nicht sichtbar ist.

Aus dieser geöffneten Ausgangslage der Fig. 3 wird nun die Verschlussklappe 5 entgegen des Pfeiles 61 um die Schwenkachse 12 geschwenkt, bis die beiden Eingriffsnuten 48, 49 der beiden Sperrelemente 45, 46 mit ihrer jeweiligen "Oberkante" am Sperrzapfen 20 anliegen. Wird nun der Spannhebel 14 durch Betätigen seines Betätigungselementes 47 um die Drehachse 15 entgegen des Pfeiles 62 gedreht, so gelangen die beiden Eingriffsnuten 48 und 49 zwangsläufig vollständig mit dem Sperrzapfen 20 in Eingriff. Bei weiterer Drehbewegung des Spannhebels 14 entgegen des Pfeiles 62 wird nun gleichzeitig die Verschlussklappe 5 entgegen des Pfeiles 61 um die Schwenkachse 12 geschwenkt. Am Ende dieser Schwenkbewegung befindet sich sowohl die Verschlussklappe 5 als auch der Spannhebel 14 in einer geschlossenen Schließposition, wie diese beispielhaft in Fig. 5 dargestellt ist.

Es ist erkennbar, dass beiden Eingriffsnuten 48, 49 mit den Sperrzapfen 20 in Eingriff stehen. Die Drehachse 15 befindet sich in dieser geschlossenen Stellung in einem geringeren Abstand zur Frontwand 17 als der Sperrzapfen 20, so dass der Behälterverschluss 4 nach dem Übertotpunktprinzip selbstständig in dieser Schließposition gehalten ist.

Diese Schließposition wird präzise durch den zweiten Anschlag 53 des Sperrelementes 46 definiert, indem an diesem Anschlag 53 außenseitig die Verschlussklappe 5 mit ihrer Lagerzunge 31 anliegt. In dieser, präzise durch den Anschlag 53 definierten Schließposition, berührt der Spannhebel 14 insbesondere mit seinem Betätigungselement 47 weder die Montageleiste 18 noch die Frontwand 17.

Wie aus der vergrößerten Darstellung der Fig. 6 ersichtlich ist, weist das Betätigungselement 47 des Spannhebels 14 insbesondere zur Montageleiste 18 einen Abstand **a** auf, welcher durch das Zusammenwirken des Anschlages 53 und der Lagerzunge 31 definiert ist.

Es ist erkennbar, dass der erfindungsgemäße Behälterverschluss 4 in einer äußerst einfachen und sicheren Weise handhabbar ist, wobei eine Beschädigung der Oberfläche sowohl der Frontwand 17 als auch der Montageleiste 18 ausgeschlossen ist. Durch die beiden Anschläge 55 und 53 ist sowohl die relative Ausgangslage des Spannhebels 14 zur Verschlussklappe 5 als auch die geschlossene Endposition des Spannhebels 14 relativ zur Verschlussklappe 5 und somit die Schließposition des Behälterverschlusses 4 präzise definiert.

Durch die Festlegung der relativen Ausgangslage des Spannhebels 14 zur Verschlussklappe 5 wird insbesondere sichergestellt, dass beim Schließvorgang, d.h. beim Schwenken der Verschlussklappe 5 entgegen des Pfeiles 61 in Schließrichtung, der Spannhebel 14 mit seinen Sperrelementen 45 und 46 bzw. der dort angeordneten Eingriffsnuten 48, 49 zwangsläufig mit dem als Sperrzapfen 20 ausgebildeten Formteil des Kupplungselementes 19 in Eingriff gelangt.

## Patentansprüche

1. Nach dem Übertotpunktprinzip arbeitender Behälterverschluss (4) zum Verriegeln eines aus einem Behälterdeckel (2) und einem kastenartigen Unterteil (3) bestehenden Sterilbehälters (1), mit einer Verschlussklappe (5), welche mit ihrem einen Ende am Behälterdeckel (2) um eine Schwenkachse (12) aus einer geöffneten Schwenkstellung in eine geschlossene Schließstellung schwenkbar gelagert ist und mit einem am anderen, freien Ende der Verschlussklappe (5) angeordneten Spannhebel (14), welcher aus einer geöffneten Ausgangsstellung in eine geschlossene Endstellung um eine Drehachse (15) an der Verschlussklappe (5) drehbar gelagert ist und, mit einem am Unterteil (3) des Sterilbehälters (1) nach außen vorstehenden Kupplungselement (19), wobei der Spannhebel (14) ein Formschlusselement (21) aufweist, welches beim Schließvorgang mit einem Formteil (20) des Kupplungselementes (19) in Eingriff bringbar ist,
**dadurch gekennzeichnet,**
**dass** ein erstes zwischen der Verschlussklappe (5) und dem Spannhebel (14) wirksames Fixierelement (55) vorgesehen ist, durch welches die geöffnete Ausgangsstellung des Spannhebels (14) relativ zur Verschlussklappe (5) derart definiert ist, dass der Spannhebel (14) während der Schließbewegung der Verschlussklappe (5) zwangläufig mit seinem Formschusselement (21) mit dem Formteil (20) des Kupplungselementes (19) in Eingriff gelangt.

2. Behälterverschluss nach Anspruch 1, **dadurch gekennzeichnet, dass** das erste Fixierelement als Anschlag (55) ausgebildet ist, welches am Spannhebel (14) oder an der Verschlussklappe (5) feststehend angeordnet ist.

3. Behälterverschluss nach Anspruch 1 oder 2, **dadurch gekennzeichnet, dass** zur Fixierung der relativen Ausgangsstellung des Spannhebels (14) zur Verschlussklappe (5) ein Rastelement (35, 36) vorgesehen ist, mit welchem der Spannhebel (14) in der Ausgangsstellung rastend in Eingriff steht.

4. Behälterverschluss nach Anspruch 3, **dadurch gekennzeichnet, dass** das Rastelement als Rastkugel (36) ausgebildet ist, welche in einem separaten Gewindezapfen (35) federelastisch nachgiebig angeordnet ist und,
dass der Gewindezapfen (35) in ein Innengewinde (34) der Verschlussklappe (5) parallel zur Schwenkachse (12) der Verschlussklappe (5) verlaufend angeordnet ist und,
dass der Spannhebel (14) ein Formschlusselement (56) aufweist, mit welchem die Rastkugel (36) rastend in Eingriff bringbar ist.

5. Behälterverschluss nach einem der Ansprüche 1 bis 4, **dadurch gekennzeichnet, dass** die geschlossene Endstellung des Spannhebels (14) relativ zur Verschlussklappe (5) durch ein zweites Fixierelement (53) derart definiert ist, dass der Spannhebel (14) in seiner geschlossenen Endstellung nicht am Unterteil (3) anliegt.

6. Behälterverschluss nach Anspruch 5, **dadurch gekennzeichnet, dass** das zweite Fixierelement als Anschlag (53) ausgebildet ist, welcher am Spannhebel (14) oder an der Verschlussklappe (5) feststehend angeordnet ist.
